# EUROPÄISCHE PATENTANMELDUNG

(11) **EP 0 737 445 A2**
(43) Veröffentlichungstag der Anmeldung: **16.10.1996**
(21) Anmeldenummer: 96111282.8
(22) Anmeldetag: 08.06.1994
(51) Int. Cl.: A61B 17/16, A61B 17/58, A61B 17/82

(54) **Zange zum Plazieren und Eindrücken eines Knochendorns in einen Knochen**

(30) Priorität: 06.07.1993 DE 4322507
(62) Teilanmeldung aus: 94920438.2
(71) Anmelder: Gundolf, Ferdinand, A-6330 Kufstein (AT)
(72) Erfinder: Gundolf, Ferdinand, A-6330 Kufstein (AT)
(74) Vertreter: Popp, Eugen, Dr.

(57) **Zusammenfassung**

Zange zum Plazieren und Eindrücken eines Knochendorns (21) in einen Knochen. Der Knochendorn (21) ist längs eines um eine Knochenfraktur herum schlingbaren Spannbandes (10) verschieblich gelagert. Vorzugsweise ist der Knochendorn (21) an der dem Knochen zugewandten Seite einer längs des Spannbandes (10) verschiebbaren Reiter-Hülse, insbesondere Flachhülse, oder eines U-förmigen Reiters (22') angeordnet. Die Zange (23) ist nach Art einer Wasserpumpenzange ausgebildet, wobei die eine Zangenbacke (24) etwa halbkreisförmig geformt ist, so daß das freie Ende (26) derselben den Knochen umgreifend der der Zugangsstelle gegenüberliegenden Seite bzw. Rückseite desselben zustellbar ist, wobei an diesem freien Ende (26) eine Halteeinrichtung (28) für den längs des den Knochen umschlingenden Spannbandes (10) versetzbaren Knochendorn (21) angeordnet ist.

## Beschreibung

Die Erfindung betrifft eine Zange zum Plazieren und Eindrücken eines Knochendorns in einen Knochen, wobei der Knochendorn längs eines um eine Knochenfraktur herum schlingbaren Spannbandes verschieblich gelagert ist.

Als Hauptziel der Frakturbehandlung gilt die Wiederherstellung der Funktion der verletzten Extremität. Zur Vermeidung von Fehlstellungen und zur Verhütung von Frakturkrankheiten (Gelenkversteifungen und Weichteilschädigungen infolge zirkulatorischer Störungen) soll dem gebrochenen Knochen durch eine stabile Osteosynthese eine Festigkeit gegeben werden, die eine länger dauernde, äußere Fixation durch Gipsverbände oder dergleichen erübrigt und eine sofortige aktive Bewegungstherapie der verletzten Extremität erlaubt. Auch bei den wiederherstellenden Eingriffen am Skelett steht neben der zuverlässigen Verknöcherung die frühzeitige aktive Funktionsbehandlung im Vordergrund. Wichtig sind ferner die Abkürzung des Krankenhausaufenthaltes, die möglichst rasche Wiederherstellung der Tragfähigkeit des Knochens und vor allem auch die Abkürzung des operativen Eingriffs. Das vorgenannte Ziel wird im wesentlichen bereits erreicht durch die Vorrichtung gemäß der DE 42 00 757 A1, die ebenfalls auf den Erfinder der vorliegenden Erfindung zurückgeht und in der Praxis erfolgreich eingesetzt wird. Dabei hat sich gezeigt, daß es günstig wäre, Maßnahmen zu ergreifen, die ein unerwünschtes Verrutschen des Spannbandes beim Umschlingen des Knochens und Spannen vermeiden. Dies läßt sich dadurch erreichen, daß längs des Spannbandes ein Knochendorn verschieblich gelagert ist, wobei der Knochendorn vorzugsweise an der dem Knochen zugewandten Seite einer längs des Spannbandes verschiebbaren Reiter-Hülse, insbesondere Flachhülse, oder eines U-förmigen Reiters angeordnet ist.

Der vorliegenden Erfindung liegt die Aufgabe zugrunde, ein Instrumentarium zur Verfügung zu stellen, welches die Handhabung des erwähnten Knochendorns erlaubt.

Diese Aufgabe wird durch eine Zange gelöst, die die Merkmale gemäß dem Kennzeichen des Anspruches 1 aufweist.

Eine vorteilhafte konstruktive Ausführungsform dieser Zange ist in Anspruch 2 beschrieben.

Nachstehend wird ein Spannband mit Knochendorn sowie eine dazugehörige Zange anhand der beigefügten Zeichnung näher erläutert. Es zeigen:
- Fig. 1: Ein mit einem längsverschieblich gelagerten Knochendorn ausgebildetes Spannband in perspektivischer Ansicht;
- Fig. 2: Eine Zange zum Plazieren und Eindrücken des beweglichen Knochendorns in den Knochen.

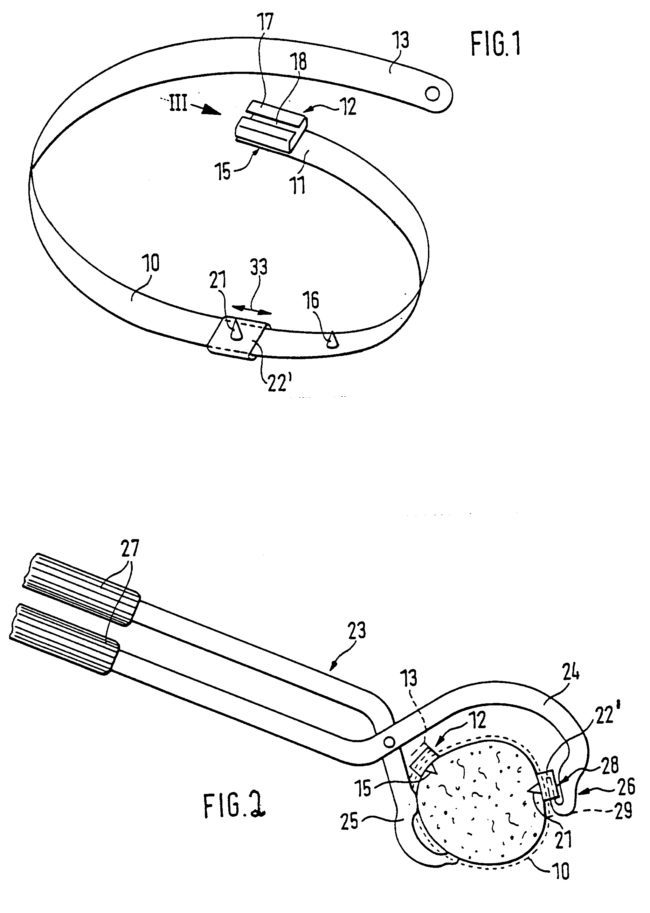

Das in Figur 1 dargestellte Spannband ist flachbandartig ausgebildet und mit der Bezugsziffer 10 versehen. An dessen einem, nämlich knochennahen Endabschnitt 11 ist ein Aufnahmeelement 12 angeordnet, durch das der andere, nämlich knochenentfernte Endabschnitt 13 des Spannbandes 10 hindurchführbar ist. Nach dem Spannen des Spannbandes um den Knochen herum wird der durch das Aufnahmeelement 12 hindurchgeführte Teil des Endabschnitts 13 zur Festlegung der eingestellten Relativlage beider Endabschnitte 11 und 13 zueinander nach oben und zurück gebogen. Auf diese Weise ist der Endabschnitt 13 des Spannbandes 10 am Aufnahmelement 12 fixiert. Diese Fixierung ist in den Figuren nicht näher dargestellt.

Das Aufnahmelement 12 ist um eine sich senkrecht zur Flachachse des Spannbandes 10 erstreckende Achse schwenkbar gelagert. Dadurch ist eine bessere Anpassung des Spannbandes an die Oberfläche eines Knochens, insbesondere an eine etwa konisch geformte Knochenoberfläche möglich. Zur Fixierung des knochennahen Endabschnitts 11 des Spannbandes 10 ist an der dem Knochen zugewandten Seite dieses Endabschnitts 11 mindestens ein in den Knochen eindringender Spike bzw. Dorn 15 (siehe auch Figur 2) angeordnet. Der Dorn 15 fluchtet mit der Schwenkachse des Aufnahmeelements 12.

Wie Figur 1 erkennen läßt, ist an der dem Knochen zugewandten Seite des Spannbandes 10 ein weiterer Spike bzw. Knochendorn 16 ausgebildet. Dieser befindet sich in der dem Aufnahmeelement 12 zugeordneten Hälfte des Spannbandes 10.

Das Aufnahmeelement 12 ist bei der dargestellten Ausführungsform durch einen zu einer Flachhülse 17 gebogenen Spannbandabschnitt gebildet, dessen freier, etwa rechteckförmiger Querschnitt dem Querschnitt des Spannbandes 10 entspricht, derart, daß der andere bzw. knochenentfernte Endabschnitt 13 des Spannbandes mit Spielpassung durch die Flachhülse 17 hindurchführbar ist. Diese Ausführungsform ist herstellungstechnisch besonders einfach. Vor allem läßt sich bei dieser Ausführungsform die Schwenkachse der Flachhulse 17 einfach fixieren. Die Flachhülse 17 ist an der Oberseite durch einen Längsschlitz 18 geteilt. Zur Fixierung der erwähnten Schwenkachse sind die beiden oberen Hälften der Flachhülse 17 etwas auseinandergebogen. Nach der Fixierung der Schwenkachse werden dann diese beiden Hälften wieder zurückgebogen, so daß die Flachhülse die in Figur 1 dargestellte Gestalt aufweist. Die Schwenkachse wird durch eine Niete gebildet, wobei der dem Knochen zugewandte Kopf eine spitzkegelige Form besitzt unter Ausbildung des erwähnten Spikes bzw. Knochendorns 15.

Die vorgenannten Bauteile sind vorzugsweise aus einer humanverträglichen Titanlegierung hergestellt.

Um die Flexibilität der beschriebenen Vorrichtung zusätzlich zu erhöhen, kann das Aufnahmeelement innerhalb vorbestimmter Grenzen relativ zur Schwenkachse kippbar sein, d. h. innerhalb vorgegebener Grenzen universalgelenkig gelagert sein. Der Kippbereich beträgt etwa 5 bis 25 Grad relativ zur Schwenkachse, und zwar allseitig. Konkret kann das vorgenannte Ziel dadurch erreicht werden, daß die Schwenkachse entweder flexibel ausgebildet, oder daß das Aufnahmeelement mit einem etwas größeren Spiel an der Schwenkachse gehalten ist.

In Figur 1 und auch Figur 2 ist noch ein weiterer Knochendorn 21 dargestellt, der auf einem U-förmigen Reiter 22' montiert längs des Spannbandes 10 verschiebbar ist. Diese Verschiebbarkeit ist in Figur 1 mit dem Doppelpfeil 33 angedeutet. Um den Reiter 22' am Spannband 10 zu halten, sind die Ränder der beiden Schenkel unter Umgreifen der beiden Längsränder des Spannbandes 10 nach innen gebogen. Statt des in Figur 1 dargestellten U-förmigen Reiters 22' kann auch eine Flachhülse vorgesehen sein, die längs des Spannbandes 10 verschiebbar ist. Der längs des Spannbandes 10 verschiebbare Knochendorn 21 ermöglicht eine individuelle Plazierung desselben. Insbesondere lassen sich mit diesem verschiebbaren Knochendorn lose Knochenfragmente fixieren. Auch läßt sich durch geeignete Plazierung des Knochendorns 21 vermeiden, daß das Spannband beim Umschlingen des Knochens und Spannen des Spannbandes in Knochenlängsrichtung verrutscht. Zu diesem Zweck wird der verschiebliche Knochendorn 21 vorzugsweise an der der Zugangsstelle durch die Fleischwunde hindurch gegenüberliegenden Seite des Knochens plaziert, so wie dies in Figur 2 dargestellt ist. Die Reiter-Hülse und der Reiter 22' sind vorzugsweise wieder aus einem Spannbandabschnitt hergestellt, auf jeden Fall aber aus dem gleichen Material wie das Spannband.

An durch die Wunde hindurch zugänglichen Stellen des Knochens empfiehlt es sich, diesen zur Plazierung eines Knochendorns 15, 16 oder 21 vorzubohren. Zu diesem Zweck kann ein Knochendorn-Bohrer dienen, der aus einem Schaft besteht, an dessen einem Ende ein Querriegel oder dergleichen und an dessen anderem Ende ein spitzkegeliger Bohrdorn angeordnet ist. Der Bohrdorn ist in der Art eines Holzbohrers ausgebildet. Der Querriegel dient als Griff zur Übertragung eines Bohrdrehmoments.

Die Plazierung des beweglichen Knochendorns 21 an der der Zugangsstelle durch die Fleischwunde hindurch gegenüberliegenden Seite des Knochens erfolgt vorzugsweise mittels einer speziellen Zange, wie sie in Figur 2 schematisch dargestellt ist. Diese Zange ist nach Art einer Wasserpumpenzange ausgebildet, wobei die eine Zangenbacke 24 etwa halbkreisförmig geformt ist, so daß das freie Ende 26 derselben den Knochen umgreifend der der Zugangsstelle gegenüberliegenden Seite zustellbar ist. An diesem freien Ende 26 ist eine Halteeinrichtung 28 für den längs des den Knochen umschlingenden Spannbandes 10 versetzbaren Knochendorn angeordnet. Die Halteeinrichtung 28 umfaßt konkret einen vom freien Ende 26 der Zangenbacke 24 weggerichteten, d. h. nach innen gerichteten Vorsprung 29 auf, auf den der versetzbare Knochendorn 21 bzw. dessen Reiter 22' aufsteckbar ist, derart, daß beim Zusammendrücken der Zangenbacken 24 und 25 Druck nur auf den Knochendorn 21, die dem Knochen zugewandte Seite des Dornreiters 22' und den zugeordneten Abschnitt des Spannbandes 10 ausgeübt wird. Auf diese Weise erfolgt keine Zusammendrückung der Reiter-Hülse durch die Zange 23. Die Zangengriffe sind mit der Bezugsziffer 27 gekennzeichnet.

## Patentansprüche

1. Zange zum Plazieren und Eindrücken eines Knochendorns (21), der längs eines um eine Knochenfraktur herum schlingbaren Spannbandes (10) verschieblich gelagert ist, wobei der Knochendorn (21) vorzugsweise an der dem Knochen zugewandten Seite einer längs des Spannbandes (10) verschiebbaren Reiter-Hülse, insbesondere Flachhülse oder U-förmigen Reiters (22') angeordnet ist, in einen Knochen,
**dadurch gekennzeichnet,** daß
die Zange (23) nach Art einer Wasserpumpenzange ausgebildet ist, wobei die eine Zangenbacke (24) etwa halbkreisförmig geformt ist, so daß das freie Ende (26) derselben den Knochen umgreifend der der Zugangsstelle gegenüberliegenden Seite bzw. Rückseite desselben zustellbar ist, wobei an diesem freien Ende (26) eine Halteeinrichtung (28) für den längs des den Knochen umschlingenden Spannbandes (10) versetzbaren Knochendorn (21) angeordnet ist.

2. Zange nach Anspruch 1,
**dadurch gekennzeichnet,** daß
die Halteeinrichtung (28) für den versetzbaren Knochendorn (21) einen vom freien Ende (26) der Zangenbacke (24) weggerichteten Vorsprung (29) aufweist, auf den der versetzbare Knochendorn (21) bzw. dessen Reiter (22') oder Reiter-Hülse aufsteckbar ist, derart, daß beim Zusammendrücken der Zangenbacken (24, 25) Druck nur auf den Knochendorn (21), die dem Knochen zugewandte Seite des Dornreiters (22') bzw. der Reiterhülse und den zugeordneten Abschnitt des Spannbandes (10) ausübbar ist.
